# EUROPEAN PATENT APPLICATION

(11) **EP 4 822 706 A1**
(43) Date of publication of application: **14.10.2026**
(21) Application number: 25169534.2
(22) Date of filing: 09.04.2025
(51) Int. Cl.: G16H 20/70

(54) **MULTI-SENSOR CALCULATION OF A MOTIVATIONAL SALIENCE INDEX**

(71) Applicant: Universite De Fribourg, 1700 Fribourg (CH)
(72) Inventor: SPIERER, Lucas, 1010 Lausanne (CH); TAPPAREL, Malika, 3971 Chermignon (CH)
(74) Representative: Schwarz, Claudia

(57) **Abstract**

The present invention relates to controlling a sensor-based craving system (100). The method for controlling a sensor-based craving system involves integrating signals from both physical sensors and data from questionnaires to assess a user's craving state. Sensors capture data during task execution, representing one aspect of the craving state, while questionnaire responses provide another. A synchronization algorithm combines these inputs into a cohesive craving dataset. This dataset informs a metric calculation algorithm, producing a motivational salience index (MSI). Calculated first and second control signals then regulate the task generator and questionnaire application respectively, enhancing the functionality of the craving system.

## Description

The present invention refers to a computer-implemented method for controlling a sensor-based craving system, a processing device and a sensor-based craving system with such an processing device and a computer program product.

Rates of overweight and obesity increased at the global and regional levels, and in all nations, between 1990 and 2021. The OECD (Organisation for Economic Co-operation and Development) found that obesity reduces GDP by an average of 3.3% in member countries due to healthcare expenses and productivity losses. A 2020 report from the World Obesity Federation estimated that obesity-related healthcare costs could reach $1.2 trillion per year by 2025 globally. Without intervention, obesity-related costs could rise by up to 300% by 2050, according to the World Health Organization (WHO). The economic impact is expected to worsen as obesity rates increase worldwide.

Further, unhealthy consumption behaviors (e.g., overeating, substance misuse, digital addictions) are often driven by powerful cravings triggered by environmental cues. However, existing solutions to monitor and manage these cravings face several critical issues:

### 1. Reliance on Self-Report Alone

Traditional methods depend heavily on users' subjective reports about their cravings and emotional states. These self-reported measures can be inconsistent or biased due to memory lapses, social desirability, or underreporting.

### 2. Lack of Objective Biomarkers

While physiological measures (e.g., heart rate, EEG, eye-tracking) can offer some objectivity, they are often costly, cumbersome to deploy, or require specialized equipment. Consequently, day-to-day craving assessments in real-world settings remain underdeveloped.

### 3. Insufficient Personalization

Many intervention programs use a "one-size-fits-all" approach, offering generic advice or standardized modules that fail to adapt dynamically to the user's specific vulnerability or fluctuating craving states.

### 4. No Closed-Loop Feedback

Existing systems typically track some user data (if at all) but rarely adjust their intervention strategies in advance or in real time. This leads to missed opportunities for immediate adapted or course-correction or targeted adjustments to current states or when cravings spike.

### 5. Ineffective Cue Identification and Management

Many digital applications address cravings superficially without pinpointing the exact cues that elevate a user's reward sensitivity. This makes it difficult to precisely target, prevent or reduce overconsumption in a timely, personalized manner.

Further, in prior art, there are approaches known, which rely on complex physiological measurements and related digital processing. The measurements are often acquired by expensive and very specialized sensors. Further, in prior art systems, determination of a person's craving state is only executable in a medical examination setting (in particular in a hospital with different types of measurement units and sensors).

Based thereon, the object of the present invention is to provide a more inexpensive and flexible solution that allows to determine a craving state without additional equipment (like expensive sensors). Further, the solution should allow for a personalized and reliable but dynamic determination of a craving state.

This object is solved by the appended claims, in particular by a (computer-implemented) method for controlling a sensor-based craving system, a processing device, and a sensor-based craving system and computer program product.

In the following, the invention will be described with respect to the claimed method first.

Features, advantages or alternative embodiments, mentioned with respect to the method can be assigned to the other claimed objects (e.g. the computer program or a processing device, or the sensor based craving system or a computer program product), and vice versa. In other words, a system, apparatus or device can be improved with features described or claimed in the context of the method, and vice versa. In this case, the functional features of the method are embodied by structural units of the apparatus or device or system and vice versa, respectively. The method may refer to a software implementation, and the device may refer to a hardware implementation (e.g. with a spatial physical structure) or a virtualization thereof. Generally, in computer science a software implementation and a corresponding hardware implementation (e.g. as an embedded system) are equivalent. Thus, for example, a method step for "receiving" data may be performed with an interface and respective instructions to read data. For the sake of avoiding redundancy, although the device may also be used in the alternative embodiments described with reference to the method, these embodiments are not explicitly described again for the device. In principle, the respective device or apparatus claim is configured to carry out the claimed method.

According to a first aspect, the invention refers to an in particular computer-implemented method for controlling a sensor-based craving system. The sensor-based craving system comprises at least a set of sensors, a questionnaire application and a task generator, both digital applications which may be implemented in hardware and/or in software. The method may comprise a step of receiving sensor signals from the set of sensors, during task execution, wherein the task has been generated by the task generator, wherein the sensor signals represent a first part of a user's craving state. The method may further comprise the step of receiving questionnaire data which are received by means of a virtual sensor in response to a provided questionnaire, wherein the questionnaire has been generated by the questionnaire application, wherein the questionnaire data represent a second part of the user's craving state. The method may continue with synchronizing received sensor signals and received questionnaire data by means of applying a synchronization algorithm to generate a synchronized craving dataset and with providing said synchronized craving dataset to a metric calculation algorithm, which is configured to calculate a motivational salience index, MSI. The method may proceed with calculating first control signals for the task generator, based on the calculated motivational salience index, MSI and with calculating second control signals for the questionnaire application, based on the calculated motivational salience index, MSI for controlling the sensor-based craving system, in particular the task generator and the questionnaire application by use of the calculated first and second control signals.

The first part of the user's craving state refers to objective sensor measurements, in particular to motion sensor data during task execution. The sensor signals may be acquired in a time phase during which the user is performing the tasks he or she has been instructed to perform. The instructions may be provided on a user interface in an optical and/or verbal form.

The second part of the user's craving state refers to subjective indications of the user, provided as reply to questions. The second part may relate to personal evaluations at the time of acquisition.

An advantage of the present invention is to be seen in that the different types of data are combined in particular for detecting a user's behavioral addiction or consumption behavior, comprising eating behavior and/or eating, tobacco consumption, alcohol consumption and or other substance consumption by means of determining his or her craving state. Detecting a user's consumption behavior or behavioral addiction by determining his/her craving state typically requires a combination of physiological, behavioral, and environmental sensors or sensor data respectively.

The method suggested herein in particular focusses on measuring cue-induced craving and/or a cue-induced MSI. Which type of cue is used is depending on the systems's setting for the person of interest. A cue might e.g., be an optical presentation of a substance or food, like providing the person with an image, representing a food item. Alternatively or in addition, a time-based cue might be considered. A time-based cue may be for example a daytime during which the person normally eats (dinner time, breakfast time etc.). Alternatively or in addition, psychological and/or emotional cues may be considered (for instance, mood-driven cravings, like stress or sadness). Alternatively or in addition, environmental and/or behavioral cues may be considered, like sensory triggers (for instance a smell, sight or sound of a substance or food). Determining a person's craving state can involve a mix of cues, emotional states, and/or environmental influences.

In particular, physical cues may be considered, like hunger signals, taste and/or nutrient deficiencies (for instance, salty cravings may indicate a need for electrolytes or dehydration or sweet cravings may indicate a drop in blood sugar or a need for quick energy) and/or time-based cues. In particular, cognitive and sensory cues may be considered, such as visual food exposure, memory-based associations, or anticipatory reward. Emotional states like boredom, anxiety, or sadness can also heighten cravings, as can time-based cues (e.g., habitual snacking at a certain hour). Unlike hunger, which is homeostatic and non-specific, cravings are typically hedonic, selective, and directed toward particular items or tastes.

In an embodiment, a cue might be provided to the user. The cue might serve as stimulus (e.g., in the form of an image of a food item). After presentation of the cue, the method may start. Thus, in this embodiment, as an initial step (preceding the other method steps), a cue is provided to the user. The cue might be provided in an optical, and/or acoustic and/or different format.

Sensor signals may comprise motion signals, which may be acquired by motion sensors. Sensor signals may alternatively or in addition comprise time measurements for completing the task. These time measurements may be acquired by means of a virtual sensor implemented on a user interface, which is configured to detect, how fast or slow a user is activating certain UI elements.

Sensor signals may comprise reaction times, force/pressure metrics, error rates, or more advanced signals (accelerometer, GSR, HRV, EEG, etc.). Sensor signals are measured during the user is executing the task. Typically sensor signals are captured via a smartphone's touchscreen/IMU or a wearable device.

Questionnaire data may refer to answers to questions, generated by the questionnaire application. Questionnaire data may comprise a personal assessment of the user. The questionnaire data may be acquired on a user interface, UI. The questionnaire data may be provided in textual form and/or in acoustic form (e.g. as voice recording) and/or as input on UI elements by means of using virtual sensors. Questionnaire data may comprise a self-report measures of craving, emotional state, or consumption patterns are administered in parallel to motor data capture, providing subjective context for the objective metrics.

Questionnaire data may comprise or may be based on subjective inputs of the user, like self-reported craving levels (scale 1-10), mood state, hunger logs, etc. The questionnaire data may preferably be entered via an app interface at discrete times. The time intervals for presenting questions to the user may be set individually and dynamically by means of the settings in the questionnaire application.

The first and/or second control signals are to be transmitted to the respective device on which the questionnaire application and/or the task generator are implemented. Depending on the deployment, all the applications may reside on the user's device, e.g. a smartphone. Alternatively or in addition, parts of the sensor-based craving system, which is to be construed as an electronic system, may be outsourced to a central computer, like a server.

The questionnaire application is configured for generating a questionnaire. A questionnaire may be construed as a set of questions. During inference, parallel to the motor tasks, a user fills out the generated questionnaire with e.g., preferences, psychological (mood, personality, etc.), and/or demographic (SES, age, gender, ...) self-report questionnaires. The generated questionnaires may be standardized or custom (user-specific). The purpose of the generated questionnaires is to gather subjective craving data, neurocognitive profiling, and/or contextual information.

Alternatively or in addition, the questionnaire application and/or the task generator is/are closed loop controlled based on the calculated MSI and/or the received sensor-based sensor signals. In particular, the questionnaire application is configured to generate the questionnaire for parameters which are also covered (detected) by the set of sensors. This has the advantage that the questionnaire data may be validated against the detected sensor data. For example, if the weight is questioned, this parameter may be validated against the measured weight of the respective weight sensor for corresponding time intervals. For this, a validation algorithm may be applied which checks the questionnaire data against the sensor-based sensor signals.

Although the calculated MSI may be displayed to the user, preferably it serves for active device adjustments as it controls the User Interface (UI) configuration. For example, if the MSI is above threshold (e.g., 70/100), the system reduces on-screen distractions, enlarges important buttons, or lowers cognitive load in tasks. If MSI is low, the app reverts to standard or higher difficulty tasks, or displays advanced modules. Further, the sensor sampling rate may be controlled based on the calculated MSI automatically. At high MSI, the device automatically increases sampling frequency for motion or physiological signals. At low MSI, sampling is reduced to conserve battery. Alternatively or in addition, the task parameters in the task generator are controlled based on the calculated MSI. For example, for Go/No-Go or Cue-Approach training: it shortens or extends reaction windows, modifies stimuli intensity, or changes the ratio of distractors vs. targets. Alternatively or in addition, the notifications and/or hardware output is controlled in response to the calculated MSI. For example, vibrations, auditory alerts, and/or LED signals are triggered if MSI surpasses a critical risk threshold. Moreover, real-time prompts push the user into a coping strategy.

Closed-loop control may involve continuous monitoring where feedback from sensors informs subsequent adjustments, creating a cycle of real-time optimization. Through these third control signals, the system can regulate various sensor parameters to enable accurate and relevant data gathering, which may optionally include control over the activation/deactivation states of sensors, adjustments to sampling rates, and modifications in data capture methodologies, such as sampling schemes.

The control of the sampling scheme influences the temporal resolution and quantity of data acquired by the sensors, affecting analysis accuracy and relevance. By customizing sampling schemes, the system adapts to varying user states or conditions, focusing sensor resources on moments or interactions important to craving assessments.

Such control invites the sensor suite to operate optimally, potentially improving the fidelity of craving-related data collected. Whether adjusting the frequency of motion sensor data capture during periods of high activity or refining virtual sensor engagement during key questionnaire points, the process aims to tailor the sensor suite's operations to align with the user's experiential landscape.

In summary, the incorporation of third control signals to manage sensor operations supports an adaptive data acquisition model within the craving system. By optionally employing closed-loop control tailored to real-time feedback, the processing device may enhance the capability of the sensors to extract meaningful and comprehensive data profiles, supporting analyses and interventions aimed at craving management.

Alternatively or cumulatively, the interface layout (functionality, size, time-related representations) may automatically adapted to accommodate the user's immediate motor/cognitive state is a device-level behavior change.

Because the data is fused in real time, the system can intervene immediately upon detecting a potential craving spike.

Hence, the invention does more than provide mental guidance-it reconfigures software/hardware elements on the user's device according to an algorithmically derived MSI.

In a preferred embodiment, a time-related modulation is used. The time-related modulation may be a time-of-day modulation or a time-phase modulation (stress time phases versus relaxed time phases or time phases over the year: winter and summer etc.). In particular, in the time-related modulation, weights and/or thresholds may be adjusted, depending on time, e.g., morning/evening usage context.

Alternatively or in addition, the sensor signals, measured during task execution may be algorithmically processed for controlling the questionnaire application - so to say without the questionnaire data -., in particular in that the sensor signals measured by the sensors trigger specific questionnaires. For instance, if a cue-induced craving based on response time or EEG signal is detected, this may automatically trigger to generate a question to be provided to the user or to use another metric to establish an association, or to validate the sensor-detected craving with a direct question.

The task generator is configured for generating tasks to be executed by the user. The tasks may comprise instructions to perform the task. The task and/or the instructions are provided on a user interface. The instructions may be provided in a visual, acoustic or other format. The task may comprise a motor task and/or a cognitive task.

The task execution is monitored by the set of sensors, like movement sensors or virtual sensors on the user interface and/or eye tracking sensors, determining the activation on UI elements (e.g., which and how fast the UI elements are activated). Eye-tracking data may in particular be acquired with a camera, in particular front camera, of a mobile device (e.g., smartphone or tablet etc.). In addition or alternatively, a camera of a desktop device may be used.

The tasks may comprise a cognitive task and/or a motor-based task (e.g., go/no-go, stimulus-response compatibility, custom motor tasks). The purpose of the generated task is to elicit user performance data under varying nature and intensity of approach-avoidance conflicts and cognitive loads.

An affective learning style may also be derived from the sensor signals measured during task execution. The affective learning style is an index that is not derived from the questionnaires, but from specific motor tasks or their execution respectively. The affective learning style is determined as the attention allocated to reward-predictive cues, measured through eye-tracking in a Pavlovian conditioning task, as well as by action choices depending on expected outcomes in a sequential reinforcement learning task.

Pavlovian conditioning means: Affective learning style in pavlovian conditioning is operationalised as the regression coefficient from a linear model predicting gaze direction based on the reward value of the conditioned stimulus. A higher coefficient reflects a stronger sign-tracking tendency, indicating greater attribution of motivational value to the cue itself.

Sequential reinforcement learning means: Affective learning bias in reinforcement learning bias is captured through the weighting parameter w, which quantifies the relative contribution of model-free (habitual) and model-based (goal-directed) systems to the computed value of an action. A lower w indicates stronger reliance on model-free learning, whereas a higher w reflects more model-based control.

The affective learning style is determined separately from receiving the sensor signals and in particular different from measuring response time or error from which cue-induced craving levels are inferred. The affective learning tasks involve motor responses but also eye-tracking. The scheme of the two affective learning bias tasks will be explained more detailed in the description of the figures.

Alternatively or in addition, the control of the sensor-based craving system is a closed loop control, in particular closed loop control of the set of sensors, in particular for controlling their sampling rate, their activation/deactivation state, and/or their transmission to the processing device, and/or closed-loop control of output devices which are configured for output of notifications (e.g., output scheme for vibration alarms, optical alarms, and/or other notification devices. This has the advantage that the set of sensors may be controlled in response to the control signals and thus in response to the calculated MSI. For instance, if the MSI is high which may represent a high craving state, it is helpful to acquire sensor signals more intensively, which means to activate sensor signal acquisition at all, to increase the sampling rate, to increiase the transmission rate etc., whereas if the MSI is low, representing a lower craving state, bandwidth and computing resources might be saved by reducing intensity of sensor signal acquisition, in particular by reducing the sampling rate or even by deactivating the sensor acquisition, and/or by reducing the transimission rate (in particular to the processing device).

Alternatively or in addition, the method may comprise the step of providing the calculated motivational salience index, MSI on a user interface and/or providing the calculated motivational salience index, MSI on an interface for further processing. This has the advantage, to increase transparency of the system and to inform the user on the outcome of the digital calculations and processings. Moreover, the MSI might be used for further downstream tasks, like adapting health parameters and/or adapting dose of pharmaceuticals and/or medical substances.

Alternatively or in addition, the synchronization algorithm may be a timestamp-based synchronization algorithm.

The timestamp-based synchronization algorithm aligns relevant motion data within a time window around T_q (e.g., [T_q - Δ, T_q]) to correlate objective and subjective signals. It ensures the contextual meaning of a craving score can be matched with the user's actual motor performance or physiological state.

By using the timestamp-based synchronization algorithm, a synchronicity in time may be provided. This ensures, that the received data, in particular at least the acquired sensor data and the questionnaire data relate to the same of similar time phase. Without precise timestamps, combining subjective and objective data in real time would be unreliable. Time alignment reduces noise, improves interpretability, and enables immediate device-level adjustments when high craving (subjective) matches certain motion anomalies (objective).

Usually, two different time sets are received: the sensor data (also sensor signals) (in particular motion sensor data during task execution) and the questionnaire data. However, optionally in addition other data sets may be received and synchronized and provided to the metric calculation algorithm to calculate the MSI. For example, other data sets may comprise physiological data acquired by physiological sensors (e.g., EEG, EKG, eye-tracking etc.) and/or hear-rate related sensors and/or glucose sensors etc. (see the options for sensors below).

Alternatively, the at least two different data sets (received sensor signals and received questionnaire data) may be integrated or synchronized independently of their time of occurrence. For instance, the synchronization algorithm may not use time to synchronize the two datasets, but other metrics, e.g., mood traits (not state) could influence cravings during a specific period of time. Mood traits may be determined by a part of the questionnaire data or by other means.

In particular, the step of controlling may comprise: controlling the task generator with the first control signals and/or controlling the questionnaire application with the second control signals. This has the advantage that the two different applications (task generator and questionnaire application) may be controlled independently and/or separately of each other. Further, this allows to only control one of the two different applications whereas the corresponding other application stays unchanged, which allows more flexibility in the control of the sensor-based craving system.

Alternatively or in addition, the synchronization algorithm may process time-stamps in the sensor signals and in the questionnaire data in order to allow a matching of the respective sensor signals with the questionnaire data, namely to match those pairs which have the same or similar timestamp. This allows to associate the two different datasets in a time domain which improves homogeneity and validity of the MSI calculation.

Alternatively or in addition, the synchronization algorithm may comprise applying a configurable weighting of the sensor signals and/or the questionnaire data for generating the synchronized craving dataset. This has the advantage that relevant data may be strengthened for MSI calculation whereas less important or relevant data may be considered less for MSI calculation. Thus, the influence of different sensor signals and/or questionnaire data may be adapted.

Alternatively or in addition, the metric calculation algorithm may use the following formula for calculating the motivational salience index, MSI: $MSI = w_m * F_m + w_q * F_q ,$ wherein
w_m and w_q are adaptive weight coefficients, F_m refers to features, extracted from the received sensor data, in particular motion data, F_q refers to the received questionnaire data which are mapped on a normalized numerical scale.

The dynamic weight coefficients are dynamically adjustable, in particular based on historical data and/or user-specific baseline measurements.

In the above formula, the sign "_m" refers to sensor signals, in particular motion signals and the sign "_q" refers to questionnaire data.

The sensor data, in particular the features extracted from the sensor data are normalized using min-max scaling or z-score normalization relative to a predefined baseline or training dataset.

The questionnaire data may be mapped to a normalized scale, such as a numerical range from 0 to 100 or 0 to 1, corresponding to predefined categories or intensity levels of subjective craving.

Adaptive weighting coefficients w_m, w_q are dynamically adjusted using statistical regression, historical calibration, or machine-learning-derived optimizations. The system may auto-refine these weights based on user outcomes. E.g., if motion patterns consistently predict high consumption episodes, w_m is increased. If self-report correlates strongly with actual relapses, w_q is boosted.

In an embodiment, a machine learning approach, ML, may be applied. The input streams are: x_m for motion features, x_q for questionnaire features. The fusion layer may comprise a concatenation or attention-based weighting across x_m and x_q. The output may comprise a scalar MSI in [0, 1] or [0, 100]. Training the machine learning method may be based on training data, comprising labeled episodes (sensor data, representing a user's state, and/or questionnaire data labeled with measured MSI based on real-world consumption outcomes). The training data are continually updated as new data arrives.

The ML approach streamlines real-time detection of craving or motivational states. Further, it may serve for data cross-checking (objective vs. subjective) reduces noise/artifact, producing higher accuracy vs. single-mode approaches.

Alternatively or in addition, the metric calculation algorithm may comprise a machine learning algorithm, in particular a neural network architecture in particular a trained form thereof. More in particular the (trained) neural network architecture may be a deep learning architecture, like a convolutional neural network, CNN, or a graph neural network, GNN or a transformer-based architecture. The sensor signals and the questionnaire data or a preprocessed form thereof are provided to an input layer of the neural network architecture. A fusion layer may be used for concatenation or attention-based weighting of the sensor signals and the questionnaire data for calculating the motivational salience index, MSI.

Preprocessing may comprise normalizing and/or standardizing the data, cleaning the data (e.g.,, handling missing data, outliers and/or duplicates), encoding (e.g., one-hot encoding for transforming categorial variables in binary vectors, label encoding and/or word embeddings for converting words into dense vector representations) and/or data augmentation. In particular data augmentation may be applied for training the neural network architecture for ensuring robustness and improving generalization.

In an inference phase (i.e., after the neural network architecture has been trained), the sensor signals and the questionnaire data or their preprocessed forms are provided as input to an input layer of the neural network.

A fusion layer, in particular an attention-based layer, concatenates or adaptively weights sensor signals and questionnaire data to produce a unified representation. The unified representation is processed by subsequent layers of the neural network to calculate the motivational salience index (MSI).

In particular, when multimodal sensor data including heart rate, and other physiological signals are acquired, a Transformer-based architecture, especially utilizing *cross-dimension and cross-time attention mechanisms such* as *described by* Zhang et al. (see: "Crossformer: Transformer Utilizing Cross-Dimension Dependency for Multivariate Time Series Forecasting", Yunhao Zhang & Junchi Yan, ICLR 2023 Conference Paper, available at https.//openreview.net/pdf?id=vSVLM2j9eie), is preferred for effectively capturing dependencies among sensor modalities and temporal dynamics.

The general flow is: Input → Attention-based fusion → CNN/GNN/Transformer layers → Output (with calculated MSI and/or control data).

Integrating machine learning gives the algorithm adaptability and scalability, encouraging the development of an MSI that reflects both immediate and nuanced motivational states. This method aims to improve craving assessment using sophisticated analytics, addressing the complexities associated with craving dynamics. Through neural architectures, the algorithm learns and adapts, capable of aligning with the user's evolving conditions, offering insights and strategies for effective craving management.

The method may aggregate and store user data over time to identify patterns, forecast risk of relapse or overconsumption, and possibly provide early alerts or targeted preemptive interventions.

According to a second aspect, the invention relates to an - in particular digital - processing device for controlling a sensor-based craving system with a set of sensors. The processing device is in data communication with a storage unit on which a questionnaire application and a task generator are implemented. The processing device may comprise:
- A sensor interface, configured for receiving sensor signals from the set of sensors during task execution, wherein the task has been generated by the task generator, wherein the sensor signals represent a first part of a user's craving state;
- A questionnaire interface, configured for receiving questionnaire data which are received by means of a virtual sensor in response to a provided questionnaire, wherein the questionnaire has been generated by the questionnaire application, wherein the questionnaire data represent a second part of the user's craving state;
- A calculator, which is configured for synchronizing received sensor signals and received questionnaire data by means of applying a synchronization algorithm to generate a synchronized craving dataset;
- wherein the calculator is further configured for providing the synchronized craving dataset to a metric calculation algorithm, which is configured to calculate a motivational salience index, MSI;
- A controller which is configured for calculating first control signals for controlling the task generator, based on the calculated motivational salience index, MSI;
- wherein the controller is further configured for calculating second control signals for controlling the questionnaire application, based on the calculated motivational salience index, MSI.

The controller may be configured to monitor the MSI in real time and to automatically adjust or trigger interventions (e.g., mindfulness prompts, coping exercises) in particular when thresholds are exceeded. The thresholds may be set in advance in a setup phase. Advantageously, the thresholds may be set individually for each user and even for different time phases, during which the user makes use of the system.

The processing device may be implemented on a server or a computing device, in particular a mobile computing device. Alternatively or in addition the processing device may at least partially be implemented on user's mobile device, e.g., on a tablet PC or a mobile phone. In particular, the user interface and the storage unit with the implemented task generator and questionnaire application may be deployed on a user-related device, in particular on a mobile device, like a mobile phone.

The processing device may be deployed on the same user-related device as the storage unit is or on another computing device, like a server. The set of sensors may comprise virtual sensors which may be located on the user interface. In addition or alternatively, the set of sensors may be located separately of a user-related device.

The controller of the processing device may comprise a control interface, for controlling the task generator and the questionnaire application.

The processing device is in data communication with the storage unit on which the questionnaire application and the task generator are implemented. The storage unit is in data communication with the user interface.

The processing device may be configured to execute the above-mentioned method for controlling the sensor-based craving system, in particular if instructed.

Overall, the processing device exemplifies a highly integrated and adaptable system architecture that can adjust task settings and questionnaire components in real-time, potentially promoting effective craving management. By combining sophisticated interfaces, algorithms, and controllers, this device operationalizes a dynamic craving modulation strategy tailored to individuals' evolving states and needs.

The sensor-based craving system is structured to encompass several advantageous components that facilitate the comprehensive monitoring and management of user cravings. This system is designed to accumulate and analyze user data within a task-oriented environment while maintaining adaptability and responsiveness to individual craving states.

At the center of the system is a set of sensors, strategically placed to capture a user's movement and/or reactions during task execution. These sensors aim to gather physical and behavioral data, translating them into signals that represent aspects of the user's craving state. Such data can reflect physiological responses, engagement levels, and reaction times, contributing to insights into craving dynamics.

The system comprises a user interface intended for direct user interaction. This interface serves as the point where users receive instructions for task execution and can offer input or feedback related to the tasks and their experiences. With this interface, users can actively engage, facilitating both the undertaking of tasks generated by the task generator and interaction with embedded applications, such as questionnaires.

A storage unit is a notable part of the system infrastructure, accommodating implementations of both a questionnaire application and a task generator. The questionnaire application supports the creation and dissemination of questionnaires, capturing subjective data that complement sensor-derived information. The task generator formulates tasks that users undertake, designed to stimulate or engage their craving dynamics, providing a structured environment for data collection.

The system is managed by a processing device that is suitable for its functionality, enabling data reception, synchronization, and analytic computation. This device is equipped with interfaces for receiving sensor signals and questionnaire data, a calculator for synchronizing these inputs, a metric calculation algorithm for determining the motivational salience index, and a controller for generating control signals to optimize both tasks and questionnaires.

Together, these components foster a sensor-based craving system that integrates continuous data acquisition, real-time interaction, and responsive modulation of user experiences. The system aligns its analytic processes to both objective and subjective measures, assisting in the nuanced management of cravings and facilitating interventions guided by up-to-date understanding of motivational states. Such a system encourages user engagement in structured settings, aiming to enhance the effectiveness and personalization of craving management approaches tailored to individual needs and conditions.

In an embodiment, the sensors are configured to detect and document the length of time it takes for a user to react to stimuli or instructions during task performance. Such reaction time data serves as a pertinent metric in assessing the engagement levels and responsiveness of users, offering indications of cognitive processing speed and possibly the intensity of craving states. This quantitative measure of user interaction can be invaluable in mapping temporal patterns that may correlate with shifts in craving magnitudes.

Moreover, the sensors are adept at capturing the movement trajectory of users throughout the task. This involves recording the pathway or motion pattern as a user navigates a physical or virtual task space. By analyzing these movement paths, the system can identify subtle behavioral cues and deviations, offering insight into the user's attentional focus and motor engagement during craving experiences. Any change in standard movement behavior can signal alterations in motivational states or identify areas where cravings might disrupt normal task performance.

This embodiment underscores the connection between hardware sensors and analytic functions in the system, ensuring every piece of captured data is not only collected but thoughtfully analyzed. This approach enhances the understanding of craving influences in real-time scenarios and allows for the fine-tuning of interventions based on rich, objective insights. Such capabilities support the precision and effectiveness of craving management strategies, tailoring them to individual user patterns and characteristics.

In another aspect the invention relates to a sensor-based craving system, comprising:
- A set of sensors, in particular for acquiring a user's movement and/or reactions during task execution;
- A user interface for user interaction and in particular for providing instructions for task execution,
- a storage unit on which a questionnaire application and a task generator are implemented and
- A processing device according to the directly preceding claim.

In this sensor-based craving system, the set of sensors may be a multimodal set of sensors in particular comprising a virtual sensor which is controlled to acquire sensor signals during task execution and/or comprising non-virtual sensors, in particular a motion sensor which is controlled to acquire motion-based sensor signals during task execution.

In the sensor-based craving system, the set of sensors may comprise a skin resistance sensor, a blood sugar sensor, in particular a subdermal blood sugar sensor, a temperature sensor, an oxygen saturation measurement device and/or electroencephalografic sensor, wherein the signals may be detected from in-ear or skin sensors and/or eye-tracking sensor, in particular embedded as camera in a mobile device.

The set of sensors may comprise physiological sensors, behavioral sensors, environmental and/or neurological sensors.

In particular, the acquired sensor data (acquired by means of the set of sensors) provide multimodal motor patterns, which are analyzed for craving state analysis.

Physiological sensors may relate to body responses. Physiological sensors detect bodily changes that may indicate hunger, craving, or consumption (e.g., eating) behavior. Physiological sensors may comprise electroencephalography sensors (EEG), electromyographical sensors (EMG), detecting muscle activity in particular in the jaw to track chewing behavior, electrodermal activity sensors (EDA) / Galvanic Skin Response (GSR),measuring skin conductivity, which can indicate emotional arousal related to cravings, and/or heart rate (HR) and/or heart rate variability sensors (HRV) (ECG, PPG), detecting changes in heart rate patterns, which can be linked to food cravings or satiety.

Alternatively or in addition, more complex or other sensors may be used, like gastrointestinal sensors (EGG - Electrogastrography), detecting stomach contractions, which indicate hunger or digestion and/or glucose monitors (CGM - Continuous Glucose Monitoring), tracking blood sugar levels to detect hunger signals.

Alternatively or in addition, eye-tracking sensors may be applied in particular during task execution. The eye-tracking sensor may preferably mounted on a user-related device, like a mobile computing device.

Behavioral sensors may be used to detect user actions and/or physical behaviors in particular during task execution. Behavioral sensors may comprise an accelerometer and/or gyroscopic sensors, detecting movements of the respective body part, like for instance, hand-to-mouth movements associated with substance consumption or eating. Alternatively or in addition, behavioral sensors may comprise smart eating tools, like smart knifes, forks and/or spoons, measuring eating speed, bite size, and chewing patterns. Alternatively or in addition, behavioral sensors may comprise a microphone, detecting user's utterances and/or sounds during task execution, like chewing sounds or swallowing to monitor eating behavior.

Generally, environmental sensors may be applied to detect context awareness as external factors influencing cravings and eating habits. Environmental sensors may comprise a proximity and/or object recognition sensor, which may be implemented as camera sensor (applied with Computer Vision, detecting user's actions and/or user interaction with food items. Alternatively or in addition, environmental sensors may comprise position sensors, like e.g., GPS, Bluetooth Beacons, detecting places linked to eating (e.g., restaurants, kitchen).

Alternatively or in addition, neurological sensors may be used, like Electroencephalography sensors (EEG), providing brainwave analysis that can reveal craving-related neural activity and/or functional Near-Infrared Spectroscopy (fNIRS), measuring brain oxygenation levels linked to craving states.

Alternatively or in addition, a scales or weight measurement unit may be used. Alternatively or in addition, the weight of the user may be provided as user input via a user interface.

An advantage of the present approach is that multiple different sensor types may be used (multimodal sensor set) which makes the MSI calculation more robust and reliable and yields the most accurate results.

Alternatively or in addition, the sensors or a least a part thereof, may be provided as part of a wearable or mobile device. For example, motion sensors of a smart watch or comparable device may be used. Alternatively or cumulatively, a camera of a mobile device may be used for eye-tracking and/or eye-gazing. Respective interfaces are provided. This has the advantage that sensor data acquisition is easy to be performed for the user. Alternatively or in addition, non-invasive sensors, are used, which makes application easier.

As the user performs the task, the set of sensors (e.g., keyboard, mouse, touchscreen, wearable as mentioned above) may record objective metrics like reaction time, pressure, motion trajectory, accuracy and/or physiological parameters (GSR, EEG, eye-tracking, HRV,..).

The sensor activation and/or sampling may be controlled dynamically during the course of applying the sensor-based craving system.

In the sensor-based craving system, the set of sensors comprises a virtual sensor, which may be implemented on the user interface and/or a motion sensor.

Alternatively or cumulatively, in the sensor-based craving system, the processing device may be configured to generate third control signals for control of the set of sensors, in particular closed loop control thereof, in particular control of a sampling scheme of the set of sensors. In a first embodiment, a uniform sampling scheme is applied to all sensors of the set of sensors. This has the advantage that the third control signals for all sensors of the set of different sensors is the same, which leads to an easy control. In a second embodiment, a distinct sampling scheme is applied to the set of sensors. With this, different control signals may be provided to the different sensors in the set of sensors. This has the advantage that the sensors may be controlled independently of each other and specifically, e.g., a high relevance sensor may be controlled with a higher sampling rate than a low relevance sensor.

Alternatively or cumulatively, in the sensor-based craving system, the questionnaire data are provided and/or processed in textual form.

In another aspect the invention relates to a computer program product comprising a computer program, the computer program being loadable into a memory unit of a computing unit, including program code sections to make the computing unit execute the method for controlling a sensor-based craving system according to an aspect of the invention, when the computer program is executed in said computing unit.

In another aspect the invention relates to a computer-readable medium, on which program code sections of a computer program are stored or saved, said program code sections being loadable into and/or executable in a computing unit to make the computing unit execute the method for controlling a sensor-based craving system according to an aspect of the invention, when the program code sections are executed in the computing unit.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

Fig. 1 shows example embodiments of a sensor-based craving system,
Fig. 2 shows an example embodiment of a sensor-based carving system in more detail,
Fig. 3 is a flow chart of a method for controlling a sensor-based craving system according to an exemplary embodiment,
Fig. 4 is a block diagram of a calculator according to an exemplary embodiment,
Fig. 5 is a block diagram of a controller according to an exemplary embodiment, and
Fig. 6 is a flow chart of a method for controlling a sensor-based craving system and
Fig. 7 is an example representation of tasks, generated by the task generator.

### DETAILED DESCRIPTION

The present invention is directed to controlling a sensor-based craving system. By fusing motor pattern analysis (during executive tasks) with questionnaire data in a multi-modal closed-loop software engine, the invention tackles several limitations in prior art simultaneously. It provides an objective, continuous, and adaptive system that identifies craving triggers, predicts high-risk consumption, and delivers tailored interventions in real time-thereby addressing the critical lack of precision and personalization found in current craving-management solutions.

This invention is a closed-loop system that quantifies and responds to a user's craving states-defined here as motivational salience-by integrating multimodal motor pattern analysis with explicit questionnaire data (e.g., demographics, preferences, emotional state). The system requires the user to perform one or more executive tasks, such as go/no-go or stimulus-response compatibility tests, during which motor metrics (including reaction time, applied force, movement trajectory, and error rates) are recorded. Simultaneously, the user completes questionnaires that capture explicit information about their preferences, emotional state, and demographic background.

A multimodal analytics engine fuses these data streams to compute a personalized Motivational Salience Index (MSI). When the MSI exceeds predetermined thresholds, the system dynamically adapts its intervention strategy in real time by delivering targeted exercises or feedback aimed at helping the user manage cravings and reduce the risk of unhealthy consumption. In summary, the invention identifies craving-inducing cues, predicts the risk of overconsumption, and provides precisely timed, adaptive interventions based on continuous analysis of both motor performance and questionnaire inputs.

Calculating the MSI, in particular by using a processing device, on which an Analytics Engine (Index Generation) may be implemented, wherein the processing device fuses motor performance data from the executive tasks with subjective questionnaire responses to calculate a Motivational Salience Index (MSI)-stands out as a key feature of this invention. By combining objective motor metrics with subjective self-reports in real time, the processing device produces a highly personalized and responsive indicator of the user's craving level. This enables the system to dynamically adjust interventions, ensuring timely and precise behavioral feedback to help mitigate cravings and reduce unhealthy consumption patterns.

The system comprises a task generator, which is configured to generate tasks. In particular, the task generator displays our outputs a cognitive or motor-based challenge (e.g., go/no-go, SRC) designed to engage the user in tasks that reveal motor performance under varying levels of cognitive load.

Existing solutions in state of the art for craving management or behavioral-change interventions typically rely on siloed data (e.g., either self-report questionnaires or limited sensor inputs), static intervention approaches, and no real-time synchronization between subjective and objective measurements.

In order to overcome the above limitations in state of the art, the present invention combines motion-related (and potentially physiological) sensor data with subjective questionnaire responses in real time and use this fused metric (the Motivational Salience Index, MSI) to automatically reconfigure a device's interface and/or sensor operations, thus producing a technical effect beyond purely psychological feedback.

Unlike known solutions, this system processes time-stamped user motion data and questionnaire inputs, algorithmically fuses them into a real-time MSI, uses the MSI as a direct control signal to adapt the device (UI, tasks, sensor sampling), demonstrating a technological improvement (e.g., optimized resource usage, real-time personalization, improved data accuracy).

Generally, time alignment reduces noise, improves interpretability, and enables immediate device-level adjustments when high craving (subjective) matches certain motion anomalies (objective).

The present invention is focused on a system and method for monitoring and controlling the cravings of a user through sensor data and questionnaire inputs. The technological field is centered around sensor technology and psychological assessment devices used to determine cravings in real time. It involves integrating multiple sensors with a questionnaire application to generate a craving state dataset that influences a feedback mechanism consisting of task generation and questionnaire adaptation. Machine learning techniques, particularly neural network architectures, may be utilized to calculate a motivational salience index, which guides the sensor-based system's control actions. This field is closely related to behavioral health interventions, human-computer interaction, and wearable technology monitoring.

Traditional solutions for assessing and managing cravings rely heavily on subjective self-reports obtained from individuals experiencing cravings. This dependence presents significant drawbacks, including susceptibility to inaccuracies stemming from memory errors, social desirability bias, or underreporting. Consequently, the data collected using these methods lack reliability and can lead to inconsistencies in craving assessment and management efforts. Furthermore, existing methodologies offer little in terms of providing objective biomarkers, which could enhance reliability and efficacy. Such approaches are often cumbersome and ill-suited for real-world applications where users require seamless integration of data collection and processing mechanisms.

The limitation of personalization within current programs is another critical shortcoming. Many systems adhere to a "One-Size-Fits-All" strategy that fails to address the unique needs, experiences, and states of individual users. This lack of person-specific adaptation may result in interventions that are less effective and engaging for the user, thereby undermining the potential success of craving management strategies. Additionally, these systems lack dynamic, real-time adaptability, offering static interventions that do not respond well to instantaneous changes in craving states, further reducing their overall effectiveness.

Moreover, existing systems are often restricted to unidirectional information flows and do not implement closed-loop feedback mechanisms. These systems fail to offer dynamic intervention adjustments based on real-time assessments and changes in the user's craving state, thus diminishing intervention precision and overall applicability. The absence of these feedback loops results in the cumbersome nature of intervention processes and limited progress in craving management.

The present inventions overcomes the above limitations by providing a multi-modal sensor based system. Key components of such system include sensor arrays that may capture biometric data (e.g., motion, skin resistance, temperature), which reflect the user's physical state during specific tasks designed to assess or manage cravings. These sensors might employ technologies familiar in biosensing, such as subdermal sensors and electroencephalographic (EEG) sensors to obtain real-time data.

Another critical component is the questionnaire application, which collects subjective data directly from the user, often treated as a virtual sensor. This synergy between objective sensor data and subjective questionnaire responses allows for a comprehensive approach to understanding craving states.

Data handling and analysis are pivotal in these systems. Algorithms, possibly employing machine learning or neural networks, are used to synthesize raw data into meaningful outputs like a motivational salience index (MSI). This index is a metric indicating the urgency or importance of a craving, guiding personalized interventions via task adjustments or changes in questionnaire methodology.

The operation of such systems requires a processing device capable of robust data management and control logic to ensure responsive and adaptive interaction with users. This indicates the application of sophisticated software engineering practices in developing interfaces and applications that cater to the dynamic nature of monitoring cravings.

The present invention aims to deliver an advanced system capable of controlling a sensor-based craving management system by leveraging sensor and questionnaire data to provide a synchronized evaluation of a user's craving state, enabling personalized and adaptive interventions.

The described solution relates to a method designed for controlling a sensor-based craving system, which combines input from a range of sources to provide an assessment and management of a user's craving states. The system includes an integrated set of sensors, a questionnaire application, and a task generator, all of which may be connected to derive insights into the motivational factors influencing user devices and users.

Moreover, the method achieves system control by utilizing the calculated control signals to govern the sensor-based craving system. This comprehensive control encompasses both the dynamic task generator and the adaptable questionnaire application, facilitating a responsive interaction model that monitors and guides the user's engagement level, craving variabilities, and motivational insights, paving the way for more informed interventions or support.

As an advantage, the system is able to adapt its interventions in real-time. Based on the MSI, the system dynamically offers targeted exercises or feedback aimed at addressing the user's current craving intensity and motivational state. This real-time adaptation can enhance user engagement and results.

Furthermore, the system's ability to capture motor data and integrate questionnaires is useful for generating the motivational salience index. Motor data might include gestures, movements, or physiological indicators, while questionnaires provide insight into the user's internal states. The synchronized craving dataset produced by these integrated inputs becomes a helpful tool for analyzing and understanding the user's craving states, enabling strategies for enhancement, support, or intervention in craving management.

Overall, this exemplary embodiment illustrates a robust system that monitors and analyzes cravings through multimodal data integration and actively adjusts interventions, including interventions on technical devices (e.g., user wearables) to suit the user's motivational context, demonstrating responsiveness and intelligence in craving management techniques.

The method incorporates a sophisticated closed-loop control mechanism, which promotes the responsiveness and adaptability of the sensor-based craving system. This closed-loop control, suitable for the set of sensors, enhances their functionality in several aspects, including the adjustment of their sampling rate, regulation of their activation/deactivation state, and management of their data transmission to a processing device. These control measures facilitate real-time adjustments based on current craving conditions, allowing the sensors to deliver relevant and timely data regarding the user's craving state.

Additionally, the method extends the closed-loop control to output devices within the system, which are configured for the delivery of notifications. These notifications may consist of feedback, guidance, or prompts intended to engage the user more deeply, address emergent cravings, or reflect progress toward their goals. The ability to control the manner and timing of notifications further personalizes the user experience, making it more effective and user-centric.

Utilizing a closed-loop approach allows the sensor-based craving system to respond dynamically to real-time data, enabling continuous monitoring and adjustment of system parameters and user notifications. By using feedback from current user states and system outputs, the closed-loop supports a cycle of sensing, analysis, adjustment, and output, assisting in a more adaptive and refined control strategy. This promotes the system's precision in managing the user's motivational salience and craving conditions, providing interventions that are timely, relevant, and effective.

Overall, this closed-loop control design encourages a robust interaction model where continuous feedback facilitates system calibration, enhancing the craving management processes. This seamless integration of sensor activity and output notifications into the closed-loop model illustrates a sophisticated control strategy aimed at sustaining user engagement and craving moderation at optimal levels, tuning responses to the user's evolving needs.

The synchronization aspect of the method may include a timestamp-based synchronization algorithm, offering an organized approach that can integrate data from various sources effectively. This algorithm can be used to align sensor signals and questionnaire data in relation to their timestamps, facilitating the generation of a coherent synchronized craving dataset.

Through employing timestamp-based synchronization, the method may achieve a refined temporal accuracy in harmonizing objective data from sensors with subjective data gathered from questionnaires. This precision could enhance the understanding of the user's craving dynamics more accurately and provide a sound foundation for analyses and interventions.

The timestamp-based synchronization algorithm assists in managing the temporal alignment of diverse datasets, potentially minimizing discrepancies from asynchronous data collection or processing events. This strategy aims to ensure that the multimodal data reflect the user's conditions comprehensively at specific points in time, allowing a real-time assessment of how different factors could interact.

Moreover, a timestamp-based approach enables systematic tracking of changes and trends over time, making craving management responsive to evolving requirements. This time-sensitive synchronization enhances the ability to analyze the user's craving condition effectively, where strategies may be adjusted accordingly, whether through recalibrated sensor settings, refined task generators, or tailored feedback notifications.

Overall, the inclusion of a timestamp-based synchronization algorithm suggests the focus on precision and reliability in capturing and combining data. This supports the sensor-based craving system's potential to deliver informed insights and actions, facilitating a nuanced understanding and efficient tracking of motivational salience states.

In the method described for managing the sensor-based craving system, the step of controlling includes specific actions that can dynamically adjust the system's functionality using tailored control signals. These control signals serve distinct purposes: first control signals for regulating the task generator and second control signals for managing the questionnaire application.

The control of the task generator using the first control signals allows for a responsive adjustment of tasks or activities that are suitable for assessing or influencing the user's craving states. By altering parameters such as difficulty, duration, or type of tasks based on the motivational salience index, the system can maintain engagement levels, address craving fluctuations, and provide challenges calibrated to the user's current condition. This strategy can promote user interest and interaction, optimizing the relevance and impact of the tasks offered.

Simultaneously, the control of the questionnaire application using second control signals facilitates refined interaction with the user, potentially improving the quality of data regarding craving states. By tailoring the type, frequency, or thematic focus of questionnaires, the system may aim to ensure that user feedback aligns closely with assessed craving conditions and needs. Such adjustments can reduce questionnaire fatigue, enhance response accuracy, and provide valuable insights into the user's subjective experiences, integrating them smoothly with objective sensor data.

This dual control process-the task generator and questionnaire application-illustrates adaptability within the craving system. By utilizing real-time data and responses to modify system components, the method encourages a closed-loop environment where exposure to stimuli, feedback mechanisms, and analysis are continuously refined. This fosters personalized engagement strategies and interventions, potentially enhancing the capacity to monitor and manage user cravings efficiently.

Ultimately, this comprehensive control integration suggests the craving system's capacity for customization and responsiveness, aligning the user's experience with their unique craving dynamics to facilitate effective craving management processes and user satisfaction.

The synchronization algorithm in the patent application is crafted to optionally integrate sensor signals and questionnaire data by processing timestamps linked with each data set. This time-focused approach is optional for matching sensor signals with questionnaire data, aiming to synchronize those with comparable timestamps, thereby seeking to enhance the temporal accuracy and relevance of the combined dataset.

One aspect of this multimodal integration may include the optional incorporation of a virtual sensor within the sensor set. The virtual sensor, if utilized, acquires sensor signals in parallel with task execution, offering an innovative method for data gathering. Unlike traditional sensors, virtual sensors have the flexibility to derive data from interactions, digital inputs, or software applications simulating real-world sensing environments. They may focus on capturing subjective elements, such as user inputs or decision-making processes, complementing the objective data collected by physical sensors.

The sensor set can also include motion sensors, acquiring motion-based signals during task execution. These motion sensors monitor users' physical activities and movements throughout task performance. This might encompass tracking fine motor actions, posture variations, or kinetic patterns that reflect engagement and distraction levels, potentially influenced by the user's craving conditions.

Together, the virtual and motion sensors form a synergistic combination, where the virtual sensor captures nuanced interaction data and the motion sensor records physical activity dynamics. The interplay of these sensors allows for a multidimensional analysis of craving states, offering a comprehensive view that aligns physiological responses with psychological and behavioral factors.

The multimodal sensor approach aids the system's aim to provide personalized feedback and interventions by offering a detailed and integrated dataset. By synchronizing the outputs of these diverse sensors, the system may identify patterns that inform craving trajectories and adjust experiences in real-time, fostering user engagement, better understanding, and effective craving modulation strategies.

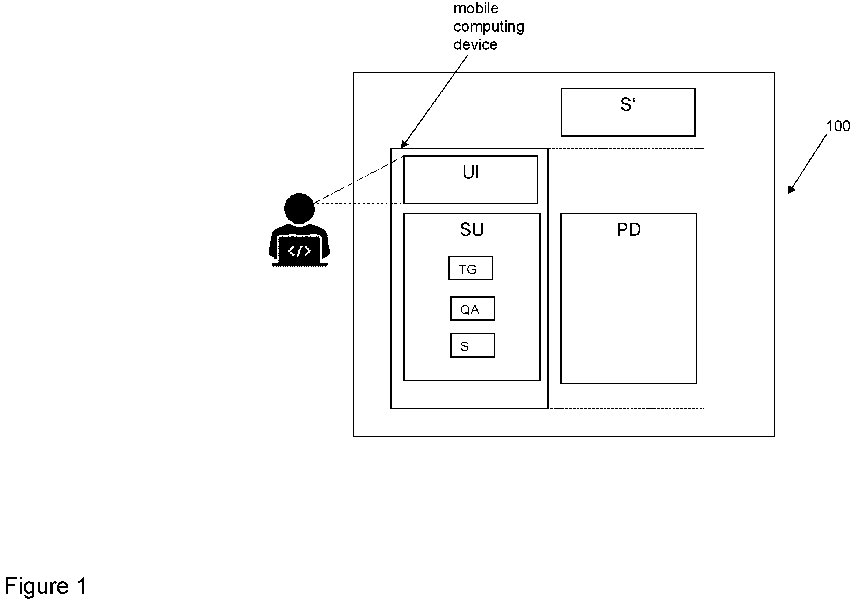

**Fig. 1** shows a sensor-based craving system (in the following also in short: system) 100 in different implementation options. The system 100 may comprise a computing device, in particular a computing device, which is associated to a user. The computing device may in particular be a mobile computing device, like a smartphone or a tablet or the like. However, it is also possible to use a personal computer.

The sensor-based craving system 100 further comprises a set of sensors S. The set of sensors may be a set of different sensors, for measuring different sensor signals of different type. The set of sensors may be deployed on the same or on different physical devices. At least some sensors of the set of sensors may be implemented on the mobile computing device, like an eye tracking sensor. In addition or cumulatively, some sensors S' may be implemented on external devices (external of the computing device) of the system 100. The sensors (including input devices, like keyboard, mouse, touchscreen, wearables) collect reaction times, force, trajectory, and/or other motor metrics while the user performs the task.

The sensor-based craving system in the present invention can optionally incorporate a diverse range of physiological and biometric sensors. These sensors aim to enhance the system's ability to gather multidimensional data relevant to user cravings, combining both surface and subdermal measurements with innovative tracking technologies.

A skin resistance sensor measures the galvanic skin response, potentially detecting variations in moisture or electrical conductivity on the skin's surface. These measurements may suggest emotional states, arousal, or stress, factors that often correlate with craving conditions. By capturing these subtle physiological changes, the sensor can provide insights into the user's internal states in relation to cravings.

A blood sugar sensor, particularly a subdermal one, offers real-time monitoring of glucose levels, relevant in understanding how physiological states influence cravings. Fluctuations in blood sugar are closely connected to cravings for specific foods or substances, offering a direct physiological marker to gauge craving intensity and its potential impact on decision-making.

Temperature sensors detect skin or core body temperature, reflecting metabolic or emotional shifts associated with various craving states. Changes in body temperature might indicate heightened states of craving or stress, aiding in constructing a comprehensive physiological profile related to craving dynamics.

An oxygen saturation measurement device assesses blood oxygen levels, providing data about respiratory function and circulatory efficiency. Variations in oxygen saturation may suggest changes in physical or emotional states, offering another metric to correlate with craving experiences.

Electroencephalographic (EEG) sensors, whether in-ear or surface skin sensors, capture brain activity, exploring cognitive processes underpinning cravings. Analyzing brainwave patterns can facilitate the exploration of attention, motivation, or decision-making processes, broadening understanding beyond purely physiological measures.

Eye-tracking technology, possibly integrated into a mobile device's camera, identifies gaze patterns and focus shifts influenced by cravings. Eye movements may reveal attentional biases and interest levels, offering psychological insights that complement physiological data.

These sensors collectively aim to allow for a profound and multidimensional analysis of craving states, integrating physiological, cognitive, and behavioral aspects. The data acquired is intended to assist in crafting personalized strategies for craving management, striving for interventions that align with the user's individual experiences and needs, leading to refined and impactful engagement.

The sensor-based craving system may integrate a virtual sensor and a motion sensor within its multimodal sensor set, potentially broadening the scope of data collection to assess craving states through diverse interaction points.

The virtual sensor could be implemented directly on the user interface, offering a method for capturing data beyond physical readings. If incorporated, this sensor can record user interactions, responses, and engagements within the digital environment. This encompasses data collection from questionnaires, task inputs, or any interactive feedback from the user. It serves as a dynamic tool suitable for simulating sensory equipment within software applications, translating user experiences into measurable data components that may contribute to understanding their craving dynamics.

The motion sensor observes users' physical activities, focusing on movements and postures during task execution. This sensor provides insights into motor responses and activity levels, potentially reflecting engagement intensity or factoring into evaluations of craving-related agitation or distraction. By using motion data, the system has the potential to analyze behavioral patterns linked to cravings, facilitating an objective look into how physical interactions correlate with affective states.

The integration of the virtual and motion sensors offers a balanced data acquisition strategy, possibly capturing both the psychological and physiological dimensions of user craving experiences. The virtual sensor observes digital habits and decision-making processes, while the motion sensor reflects physicality and kinesthetic engagement. Together, they may provide a multi-layered perspective beneficial for understanding and managing cravings.

This combined sensor utility aims to enhance the craving system's responsiveness and adaptability, potentially using real-time data to inform how cravings manifest and how interventions could be tailored. With both virtual and physical perspectives, the system strives to enrich personalized craving management, optimizing strategies to suit individual user needs and circumstances, fostering engagement and possibly mitigating cravings through adaptive techniques.

The sensor-based craving system benefits from a processing device that is capable of generating third control signals suitable for managing the set of sensors, optionally through a closed-loop control mechanism. This functionality allows for dynamic regulation of sensor operations, potentially enhancing the system's responsiveness and precision in data collection.

The system 100 further comprises a processing device PD and a storage unit SU and a user interface UI. In a first exemplary embodiment shown in Fig.1 in solid line, the computing device may comprise the user interface UI and the storage unit, whereas the processing device PD is deployed somewhere else on another device, separate from the computing device. In a second exemplary embodiment shown in Fig. 1 in dotted line, the computing device may also comprise the processing device PD as well.

The storage unit SU may be a memory. The storage unit SU may comprise a task generator TG, a questionnaire application QA and optionally at least a part of the set of sensors S.

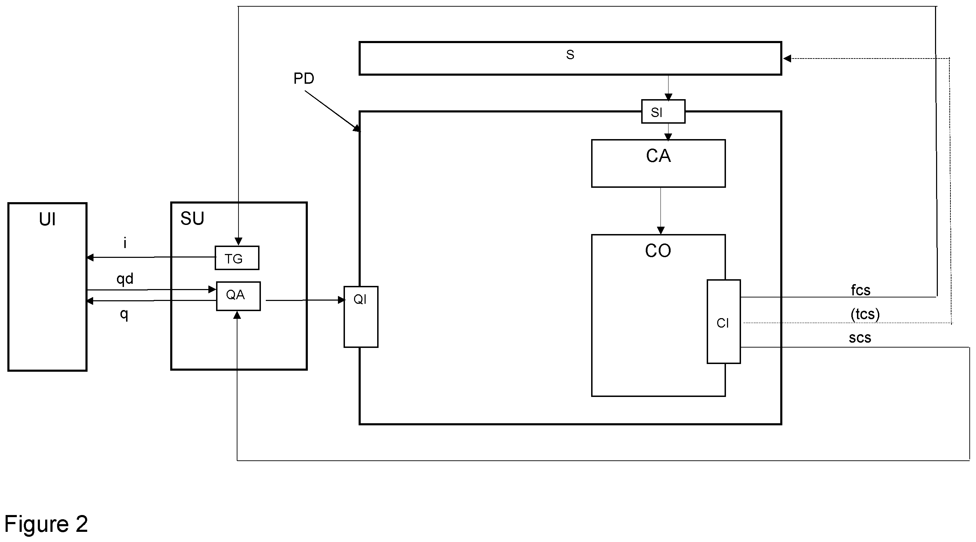

**Fig.** 2 shows a more detailed representation of the system 100 in other possible embodiments. The system 100 may comprise the user interface UI, the processing device PD, the storage unit SU and a set of sensors S.

In the storage unit SU the task generator TG and the questionnaire application QA are stored and deployed. In a preferred embodiment, the storage unit SU may be directly implemented as internal storage of the processing device PD. The user interface UI may also be implemented as part of the processing device PD. In this case, the respective interfaces are internal interfaces. However, in other deployments, the before mentioned components may be deployed differently and/or externally. The processing device PD comprises the calculator CA and the controller CO.

The processing device PD may comprise a sensor interface SI, configured for receiving sensor signals from the set of sensors S during task execution. The task has been generated by the task generator TG. The sensor signals represent a first part of a user's craving state.

The processing device PD may comprise a questionnaire interface QI, configured for receiving questionnaire data which are received by means of a virtual sensor in response to a provided questionnaire. The questionnaire has been generated by the questionnaire application QA. The questionnaire data represent a second part of the user's craving state.

The calculator CA is configured for synchronizing received sensor signals and received questionnaire data by means of applying a synchronization algorithm. After applying the synchronization algorithm, a synchronized craving dataset is generated or provided.

The calculator CA is further configured for providing the synchronized craving dataset to a metric calculation algorithm S5. The metric calculation algorithm S5 is configured to calculate a motivational salience index, MSI.

The controller CO is configured for calculating first control signals fcs for controlling the task generator TG, based on the calculated motivational salience index, MSI, as depicted in Fig. 2 with the arrow.

The controller CO is further configured for calculating second control signals scs for controlling the questionnaire application QA, based on the calculated motivational salience index, MSI, also depicted with the arrow in Fig. 2.

Optionally, the controller CO may be configured for calculating third control signals tcs for controlling the set of sensors S. As this is optional, it is depicted in Fig. 2 with dotted line.

The controller CO may comprise a control interface CI for transmitting the control signals fcs, scs, tcs to the respective components and/or applications.

The controller CO may comprise a questionnaire interface QI for receiving questionnaire data qd. In Fig. 2 the questionnaire data qd are received from the questionnaire application. However, preferably, the questionnaire data qd are received directly from the user interface UI. The questionnaire data qd may be (buffer) stored in the storage unit SU, in particular triggered by the questionnaire application QA.

The first control signals fcs are transmitted to the task generator TG. Based on the first control signals fcs, the task generator TG is triggered or activated to generate tasks. For this purpose, instructions i may be issued to be provided on the user interface UI. The instructions i may be directed to a user. For example, the instructions i may serve to execute a cognitive task and/or motor task (e.g., raise your hand).

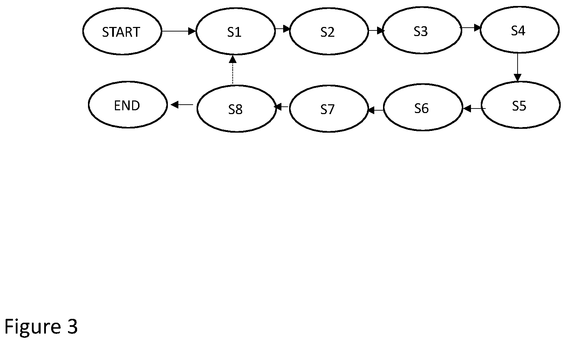

**Fig. 3** is a flow char of a method for controlling the sensor-based craving system 100. The sensor-based craving system 100 comprises the set of sensors S, the questionnaire application QA and the task generator TG. After start of the method, in step S1 sensor signals are received from the set of sensors S, during task execution. The task has been generated by the task generator TG. The sensor signals represent a first part of a user's craving state. In step S2, which may be executed subsequently to step S1 or in parallel to step S1, questionnaire data are received by means of a virtual sensor in response to the provided questionnaire. The questionnaire has been generated by the questionnaire application QA.

The questionnaire data represent a second part of the user's craving state. In step S3, the received sensor signals and the received questionnaire data are synchronized by means of applying a synchronization algorithm to generate a synchronized craving dataset. In a simple form, the synchronization algorithm may fuse or concatenate the two different data sets (sensor signals and questionnaire data). In a more complex form, the synchronization algorithm may match the items in the two different datasets based on their acquisition or generation time. In step S4, the synchronized craving dataset is provided to a metric calculation algorithm S5, which is configured to calculate the motivational salience index, MSI. In step S6, first control signals fcs for controlling the task generator TG are generated, based on the calculated motivational salience index, MSI. In step S7, second control signals scs for controlling the questionnaire application QA are generated, based on the calculated motivational salience index, MSI. Finally, in step S8, the sensor-based craving system 100, in particular the task generator TG and the questionnaire application QA is controlled by use of the calculated first and second control signals fcs, scs. After this, the method may end or may be re-iterated as sown in Fig. 3 with dashed line.

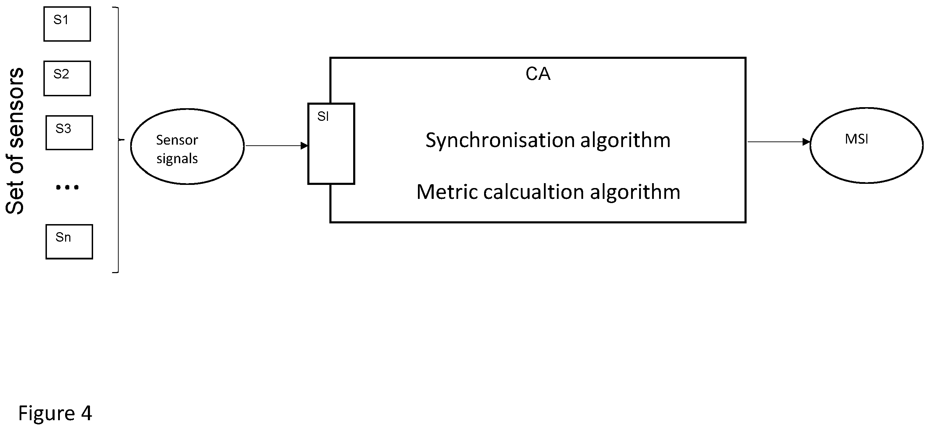

**Fig. 4** is a schematic representation of a calculator CA. On the calculator CA, the synchronization algorithm and the metric calculation algorithm may be deployed. The set of sensors S may comprise a plurality of sensors of different type, and/or wherein the sensors of the set of sensors may be positioned in different components or devices. So, a first sensor may be deployed on the mobile device, e.g., a user mobile device, like a smartphone, and a second sensor may be deployed on a first wearable and a third sensor may be deployed on as implanted sensor etc. The set of sensors acquire sensor signals which are received on the calculator CA via the sensor interface SI. The calculator CA is configured to calculate or determine the MSI.

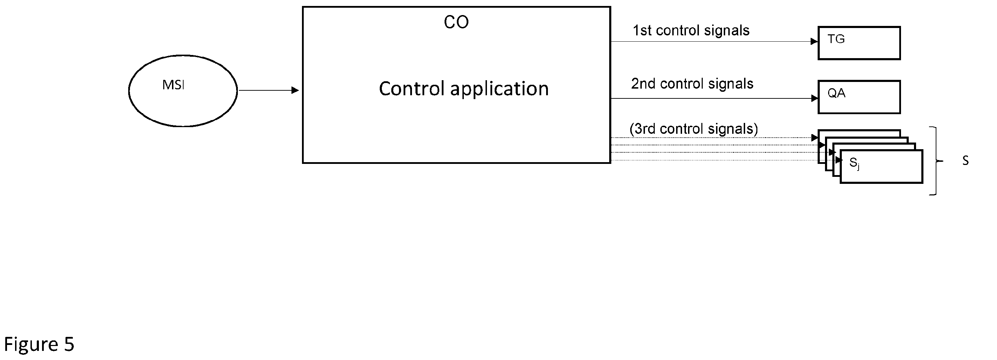

**Fig. 5** is a schematic representation of a controller CO. On the controller CO, a controller application may be deployed, the controller application may be configured to calculate or determine control signals fcs, scs, tcs based on the calculated MSI. As depicted in Fig. 5, the third control signals tcs, are optional, depicted in dotted lines.

Alternatively or cumulatively, the third control signals tcs may be generated specifically for each sensor or each sensor type separately. With this, the different sensors or sensor types may be controlled independently of each other. This means, that a virtual sensor, e.g. on the user interface, may be controlled differently than a physical sensor, like the temperature sensor or than the blood sugar sensor.

A virtual sensor may be construed as a software-based system that estimates or simulates the behavior of at least one physical sensor. It may use mathematical models, machine learning, or data from other measurements and process parameters to calculate the desired quantity or predict what a physical sensor would measure. Virtual sensors may be employed when physical sensors are impractical due to cost, spatial constraints, or invasiveness for the user.

Generally, the control of physical sensors involves managing various aspects to ensure accurate and reliable data collection and proper integration within the system 100. In particular, controlling a sensor may comprise
- adjusting the sensor to ensure its output corresponds accurately to the measured physical quantity, such as temperature, pressure, or motion;
- modifying how responsive the sensor is to changes in the measured parameter. This is crucial for applications requiring high precision or noise reduction;
- controlling how frequently the sensor takes measurements. Higher sampling rates are used for dynamic systems, while lower rates may suffice for static environments;
- setting the operational range of the sensor to ensure it can measure within the expected limits of the application without saturation or damage;
- applying signal processing techniques to filter out noise and improve the quality of the sensor's output signal and/or
- managing power consumption by turning sensors on/off or putting them into low-power modes when not in use, especially in battery-operated systems;
- configuring how the sensor communicates with the system and in particular with the processing device, such as selecting between analog or digital outputs and setting up protocols like I2C, SPI, or UART;
- defining thresholds for triggering alerts or actions when specific conditions are met (e.g., heart rate exceeding a safe limit); and/or
- monitoring the sensor's operational status to detect faults or degradation over time, ensuring consistent performance.

For all or some of these control tasks, the respective control signals may be provided.

These controls enhance the efficiency, accuracy, and reliability of the (physical) sensors.

In particular, control of the sensor may refer to its sampling rate and/or its transmission rate to the processing device PD.

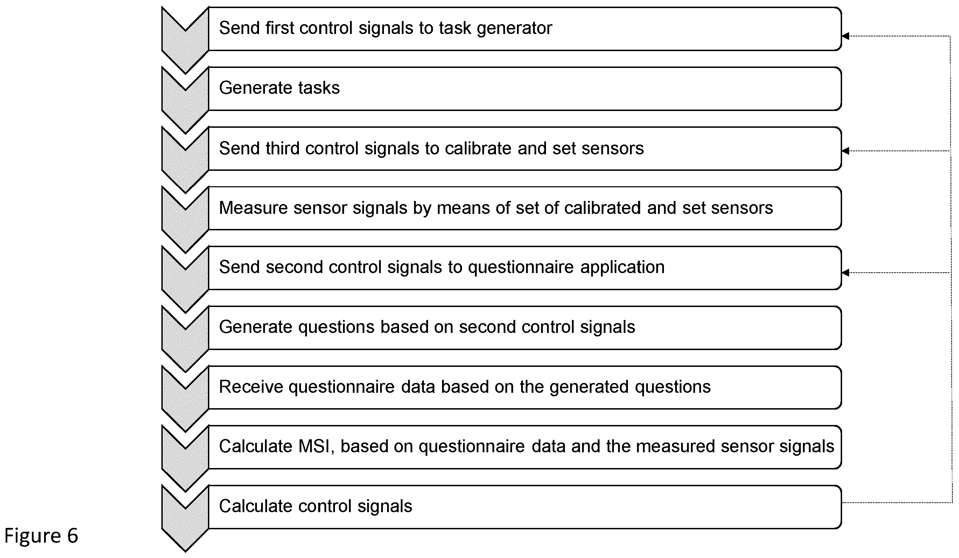

**Fig. 6** is a flowchart of the method of controlling the system 100. The first control signals fcs are sent to the task generator TG. After the first control signals fcs are received, the task generator TG generates the tasks. The third control signals tcs are sent to the set of sensors to calibrate and/or set the sensors of the set of sensors S. After calibration and/or setting of the sensors in the set of sensors S, the sensors are configured to measure the sensor signals.

The second control signals scs are sent to the questionnaire application QA. Based on receiving the second control signals scs, the questions are generated. The generated questions may be output on the user interface UI. After this, the questionnaire data qd may be received. After having received the measured sensor signals and the questionnaire data, the MSI is calculated based thereon. After having calculated the MSI, the control signals may be generated and provided to the respective devices (like sensors or computing device). The sequence in which the first, second and third control signals are transmitted may be altered.

This should be represented in Fig. 6 with the dotted back arrows. It is also possible to only use a subset of these control signals for the step of controlling.

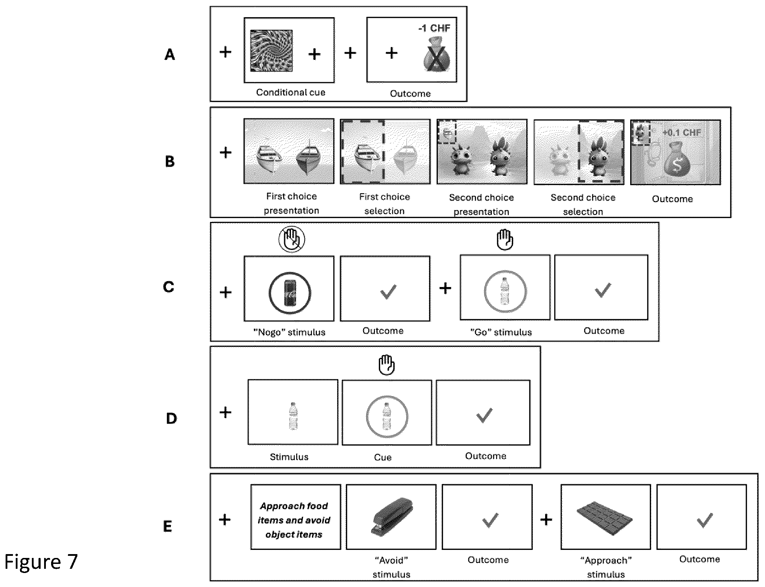

**Fig. 7** is an example representation of tasks, which may be generated by the task generator TG.

### A) pavlovian conditioning (affective learning bias measure)

The Pavlovian conditioning task involves learning associations between visual cues (fractal images) and monetary outcomes, ranging from losses to gains. It allows to identify participants' affective learning profiles in Pavlovian learning by measuring gaze patterns for different cue-outcome associations, using eye-tracking data.

Sequence: Stimulus (cue) presentation → Monetary outcome → Eye-tracking response.

### B) sequential learning task: (affective learning bias measure)

The sequential learning task involves two-step decision-making across probabilistic transitions and rewards, requiring participants to adapt their choices based on changing outcomes. It is used to assess affective learning bias in reinforcement learning by modelling the balance between reinforcement learning strategies through computational analysis.

Sequence: First-stage stimulus → First choice → Second-stage stimulus (after transition) → Second choice → Outcome (reward or not) → Learning update.

### C) go/nogo (GNG): (Motor proxy of reward system activity)

The Go/NoGo training task consists of repeatedly responding to certain items (Go) while withholding responses to others (NoGo), creating an automatic motor inhibition. It reduces the perceived value of specific items (e.g., unhealthy foods) by consistently pairing them with response inhibition, thereby supporting behavioural change.

Sequence: Item presentation → Go/NoGo cue → Motor response or inhibition → Repetition across trials → Value modulation.

### D) cue approach training: (Motor/attentional proxy of reward system activity)

The Cue Approach Training task presents food items on-screen, prompting participants to respond quickly only when a visual and auditory cue appears, reinforcing timely reactions with positive feedback. It reduces the salience of trained items, thereby supporting behavioral change.

Sequence: Food item presentation → Cue (on subset of trials) → Speeded response (keypress) → Positive feedback → Change in salience.

### E) mobile-AAT: (Motor/ attentional proxy of reward system activity)

The Mobile Approach-Avoidance Task (Mobile AAT) measures participants' automatic approach bias toward high-calorie foods in real-life settings using smartphone-based motion responses. It is personalized based on participants' food preferences and assesses implicit tendencies by comparing reaction times for approaching vs. avoiding food and control stimuli.

Sequence: Item presentation on smartphone → Swipe toward/away (approach/avoid) → Motion response recorded → Reaction time analyzed → Implicit bias measured.

In summary, the focus of the present invention is to combine and fuse sensor signals from physical and/or virtual sensors and questionnaire data for calculating thereon an MSI. The MSI is further processed to control the sensor-based craving system with its devices.

## Claims

1. Method for controlling a sensor-based craving system (100), wherein the sensor-based craving system (100) comprises a set of sensors (S), a questionnaire application (QA) and a task generator (TG), wherein the method comprises the following method steps:
- Receiving (S1) sensor signals from the set of sensors (S), during task execution, wherein the task has been generated by the task generator (TG), wherein the sensor signals represent a first part of a user's craving state;
- Receiving (S2) questionnaire data which are received by means of a virtual sensor in response to a provided questionnaire, wherein the questionnaire has been generated by the questionnaire application (QA), wherein the questionnaire data represent a second part of the user's craving state;
- Synchronizing (S3) received sensor signals and received questionnaire data by means of applying a synchronization algorithm to generate a synchronized craving dataset;
- Providing (S4) the synchronized craving dataset to a metric calculation algorithm (S5), which is configured to calculate a motivational salience index, MSI;
- Calculating (S6) first control signals (fcs) for controlling the task generator (TG), based on the calculated motivational salience index, MSI;
- Calculating (S7) second control signals (scs) for controlling the questionnaire application (QA), based on the calculated motivational salience index, MSI;
- Controlling (S8) the sensor-based craving system, in particular the task generator (TG) and the questionnaire application (QA) by use of the calculated first and second control signals (fcs, scs).

2. Method according to claim 1, wherein the control of the sensor-based craving system (100) is a closed loop control, in particular closed loop control of the set of sensors (S), in particular for controlling their sampling rate, their activation/deactivation state, and/or their transmission to the processing device, and/or closed-loop control of output devices which are configured for output of notifications.

3. Method according to any of the preceding claims, wherein the synchronization algorithm is a timestamp-based synchronization algorithm.

4. Method according to any of the preceding claims, wherein the step of controlling (S8) comprises: controlling the task generator (TG) with the first control signals (fcs) and/or controlling the questionnaire application (QA) with the second control signals (scs).

5. Method according to any of the preceding claims, wherein the synchronization algorithm processes time-stamps in the sensor signals and in the questionnaire data to match the respective sensor signals with the questionnaire data, which have the same or similar timestamp and/or wherein the synchronization algorithm comprises to apply a configurable weighting of the sensor signals and/or the questionnaire data for generating the synchronized craving dataset.

6. Method according to any of the preceding claims, wherein the metric calculation algorithm (S5) uses the following formula for calculating the motivational salience index, MSI: $MSI = w_m * F_m + w_q * F_q ,$
wherein
w_m and w_q are adaptive weight coefficients, F_m refers to features, extracted from the received sensor data, in particular motion data, F_q refers to the received questionnaire data which are mapped on a normalized numerical scale.

7. Method according to any of the preceding claims, wherein the metric calculation algorithm (S5) comprises a machine learning algorithm, in particular a neural network architecture, in particular a deep learning architecture, like a convolutional neural network, CNN, or a graph neural network, GNN or a transformer-based architecture by providing the sensor signals and the questionnaire data or a preprocessed form thereof to an input layer and using a fusion layer for concatenation or attention-based weighting of the sensor signals and the questionnaire data for calculating the motivational salience index, MSI.

8. Processing device (PD) for controlling a sensor-based craving system (100) with a set of sensors (S), wherein the processing device (PD) is in data communication with a storage unit (SU) on which a questionnaire application (QA) and a task generator (TG) are implemented, wherein the processing device (PD) comprises:
- A sensor interface (SI), configured for receiving (S1) sensor signals from the set of sensors (S) during task execution, wherein the task has been generated by the task generator (TG), wherein the sensor signals represent a first part of a user's craving state;
- A questionnaire interface (QI), configured for receiving (S2) questionnaire data which are received by means of a virtual sensor in response to a provided questionnaire, wherein the questionnaire has been generated by the questionnaire application (QA), wherein the questionnaire data represent a second part of the user's craving state;
- A calculator (CA), which is configured for synchronizing (S3) received sensor signals and received questionnaire data by means of applying a synchronization algorithm to generate a synchronized craving dataset;
- wherein the calculator (CA) is further configured for providing (S4) the synchronized craving dataset to a metric calculation algorithm (S5), which is configured to calculate a motivational salience index, MSI;
- A controller (CO) which is configured for calculating (S6) first control signals (fcs) for controlling the task generator (TG), based on the calculated motivational salience index, MSI;
- wherein the controller (CO) is further configured for calculating (S7) second control signals (scs) for controlling the questionnaire application (QA), based on the calculated motivational salience index, MSI.

9. A sensor-based craving system (100), comprising:
- A set of sensors (S), in particular for acquiring a user's movement and/or reactions during task execution;
- A user interface (UI) for user interaction and in particular for providing instructions (i) for task execution,
- a storage unit (SU) on which a questionnaire application (QA) and a task generator (TG) are implemented and
- A processing device (PD) according to the directly preceding claim.

10. The sensor-based craving system (100) according to the directly preceding claim, wherein the set of sensors (S) is a multimodal set of sensors in particular comprising a virtual sensor which is controlled to acquire sensor signals during task execution and/or comprising a motion sensor which is controlled to acquire motion-based sensor signals during task execution.

11. The sensor-based craving system (100) according to any of the preceding system claims, wherein the set of sensors (S) comprises a skin resistance sensor, a blood sugar sensor, in particular a subdermal blood sugar sensor, a temperature sensor, an oxygen saturation measurement device and/or electroencephalografic sensor, wherein the signals may be detected from in-ear or skin sensors and/or eye-tracking sensor, in particular embedded as camera in a mobile device.

12. The sensor-based craving system (100) according to any of the directly preceding system claims, wherein the set of sensors (S) comprises a virtual sensor, which may be implemented on the user interface and/or a motion sensor.

13. The sensor-based craving system (100) according to any of the directly preceding system claims, wherein the processing device (PD) is configured to generate third control signals (tcs) for control of the set of sensors, in particular closed loop control thereof, in particular control of a sampling scheme.

14. The sensor-based craving system (100) according to any of the directly preceding system claims, wherein the questionnaire data are provided and/or processed in textual form.

15. Computer program product being loadable into a memory unit of a computing unit, including program code sections to make the computing unit execute the method for controlling a sensor-based craving system (100) according to a method as claimed in any of the preceding method claims, when the computer program is executed in said computing unit.
